# EUROPEAN PATENT APPLICATION

(11) **EP 4 119 050 A1**
(43) Date of publication of application: **18.01.2023**
(21) Application number: 21769006.4
(22) Date of filing: 03.03.2021
(51) Int. Cl.: A61B 5/11, A61B 5/16

(54) **SIGNAL PROCESSING SYSTEM, SENSOR SYSTEM, LIVING BODY MANAGEMENT SYSTEM, ENVIRONMENT CONTROL SYSTEM, SIGNAL PROCESSING METHOD, AND PROGRAM**

(30) Priority: 11.03.2020 JP 2020042356
(71) Applicant: Panasonic Intellectual Property Management Co., Ltd., Osaka-shi, Osaka 540-6207 (JP)
(72) Inventor: SUZUKA, Yuko, Osaka-shi, Osaka 540-6207 (JP); WAKABAYASHI, Toshikazu, Osaka-shi, Osaka 540-6207 (JP)
(74) Representative: Appelt, Christian W.
(86) International application number: PCT/JP2021/008239
(87) International publication number: WO 2021/182250

(57) **Abstract**

The problem to be overcome by the present disclosure is to provide a signal processing system, a sensor system, a biometric management system, an environmental control system, a signal processing method, and a program, all of which contribute to recognizing the condition of a person's autonomic nervous system even without using a high-accuracy heartbeat sensor. A signal processing system (2) includes a heartbeat information acquisition unit (21), a representative value calculation unit (23), and a nerve recognition unit (25). The heartbeat information acquisition unit (21) acquires, from a radio wave sensor (1), heartbeat information as a result of detection of a person's (HI) heartbeat. The representative value calculation unit (23) calculates, based on the heartbeat information, a representative value of an interval of the heartbeat during a predetermined period. The nerve recognition unit (25) recognizes, based on the representative value, a condition of the person's (HI) autonomic nervous system.

## Description

### Technical Field

The present disclosure relates to a signal processing system, a sensor system, a biometric management system, an environmental control system, a signal processing method, and a program.

### Background Art

A mental activity determination apparatus is known in the art as an apparatus for determining a human mental activity by using the fact that there is correlation between the result of a frequency analysis on heartbeat interval data and the respective degrees of activity of the sympathetic nervous system and the parasympathetic nervous system (see, for example, Patent Literature 1). The mental activity determination apparatus detects the heartbeat interval and calculates a variance of the heartbeat interval data thus detected and the average value of the heartbeat interval or the heart rate. The mental activity determination apparatus determines the subject's mental activity based on the variance of the heartbeat interval and either the average heartbeat interval or the average heart rate.

The mental activity determination apparatus uses an electrocardiogram detector for measuring the subject' electrocardiogram signal in order to detect the heartbeat interval. In this case, the subject wears electrodes for detecting a potential signal. Alternatively, a pulse wave sensor is attached to one of the subject's ears or hands to detect the pulse wave.

However, to detect a person's (subject's) heartbeat interval as in the mental activity determination apparatus described above, a high-accuracy heartbeat sensor such as an electrocardiogram detector needs to be used.

### Citation List

### Patent Literature

Patent Literature 1: JP H08-280637 A

### Summary of Invention

It is therefore an object of the present disclosure to provide a signal processing system, a sensor system, a biometric management system, an environmental control system, a signal processing method, and a program, all of which are configured or designed to recognize the condition of a person's autonomic nervous system even without using a high-accuracy heartbeat sensor.

A signal processing system according to an aspect of the present disclosure includes a heartbeat information acquisition unit, a representative value calculation unit, and a nerve recognition unit. The heartbeat information acquisition unit acquires, from a radio wave sensor, heartbeat information as a result of detection of a person's heartbeat. The representative value calculation unit calculates, based on the heartbeat information, a representative value of an interval of the heartbeat during a predetermined period. The nerve recognition unit recognizes, based on the representative value, a condition of the person's autonomic nervous system.

A sensor system according to another aspect of the present disclosure includes the signal processing system described above and a radio wave sensor that receives a radio wave reflected from the person. The radio wave sensor detects the heartbeat information and outputs the heartbeat information to the signal processing system.

A biometric management system according to still another aspect of the present disclosure includes the sensor system described above and a biometric condition determination unit that determines, based on a result of recognition made by the nerve recognition unit, the person's mental and/or physical condition(s) as a biometric condition.

An environmental control system according to yet another aspect of the present disclosure includes the biometric management system described above and an equipment controller that controls, based on a decision made by the biometric condition determination unit, equipment for changing an environment in a space where the person is present.

A signal processing method according to yet another aspect of the present disclosure includes a heartbeat information acquisition step, a representative value calculation step, and a nerve recognition step. The heartbeat information acquisition step includes acquiring, from a radio wave sensor, heartbeat information as a result of detection of a person's heartbeat. The representative value calculation step includes calculating, based on the heartbeat information, a representative value of an interval of the heartbeat during a predetermined period. The nerve recognition step includes recognizing, based on the representative value, a condition of the person's autonomic nervous system.

A program according to yet another aspect of the present disclosure is designed to cause a computer system to perform the signal processing method described above.

### Brief Description of Drawings

FIG. 1 is a block diagram illustrating a sensor system including a signal processing system according to an exemplary embodiment;
FIG. 2 is a waveform chart showing exemplary time series data of an average heartbeat interval obtained by the sensor system;
FIG. 3 is a block diagram illustrating a biometric management system according to the exemplary embodiment;
FIG. 4 is a block diagram illustrating an environmental control system according to the exemplary embodiment;
FIG. 5 is a perspective view illustrating an exemplary arrangement of pieces of equipment in the environmental control system; and
FIG. 6 is a flowchart showing the procedure of a signal processing method according to the exemplary embodiment.

### Description of Embodiments

The exemplary embodiment to be described below generally relates to a signal processing system, a sensor system, a biometric management system, an environmental control system, a signal processing method, and a program. More particularly, the exemplary embodiment relates to a signal processing system, a sensor system, a biometric management system, an environmental control system, a signal processing method, and a program, all of which are configured or designed to recognize the condition of a person's autonomic nervous system.

Note that the embodiment to be described below is only an exemplary one of various embodiments of the present disclosure and should not be construed as limiting. Rather, the exemplary embodiment may be readily modified in various manners depending on a design choice or any other factor without departing from the scope of the present disclosure.

### (Embodiment)

### (1) Overview of sensor system

It is well known in the art that it is useful to recognize the condition of a person's autonomic nervous system in order to determine his or her mental and/or physical condition(s) as a biometric condition.

Specifically, an LF/HF ratio is used as an index to the condition of a person's autonomic nervous system. The LF/HF ratio may be obtained based on time series data of RRI (RR interval). As used herein, the "RRI" refers to a time interval between one R wave and the next R wave on a person's electrocardiogram, i.e., the person's heartbeat interval (hereinafter also referred to the "interval of the heartbeat"). The RRI constantly varies, and the distribution of the varying frequency of the RRI may be calculated as a power spectrum based on the time series data of the RRI In the power spectrum, LF is an integral value of signal strengths in a low-frequency domain (from 0.05 Hz to 0.15 Hz, for example) and HF is an integral value of signal strengths in a high-frequency domain (from 0.15 Hz to 0.40 Hz, for example). The LF/HF ratio when a person's autonomic nervous system is sympathetic dominant is larger than the LF/HF ratio when the person's autonomic nervous system is parasympathetic dominant. For example, the more stressed a person feels, the larger the LF/HF ratio is. The more relaxed a person feels, the smaller the LF/HF ratio is.

To obtain the RRI, however, a person's heartbeat needs to be detected with high accuracy. Examples of high-accuracy heartbeat sensors which are currently used in practice include a contact-type sensor for making evaluation either based on an electrocardiogram or by an optical method. However, enabling recognizing the condition of a person's autonomic nervous system without using such a high-accuracy heartbeat sensor would broaden the range of sensors available for use and improve the system versatility. Also, enabling recognizing the condition of a person's autonomic nervous system without making contact with his or her body would make him or her feel much less constrained by wearing the sensor on him or her, which would relieve his or her mental and/or physical burden.

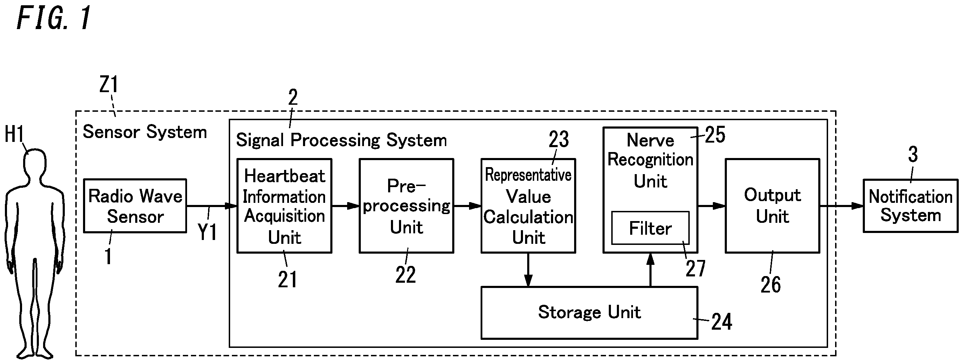

FIG. 1 illustrates, as a sensor system according to an exemplary embodiment, a configuration for a sensor system Z1 that enables recognizing the condition of a person's autonomic nervous system without using a high-accuracy heartbeat sensor. The sensor system Z1 includes a radio wave sensor 1 and a signal processing system 2.

### (2) Radio wave sensor

The radio wave sensor 1 detects heartbeat information as a result of detection of a person's H1 heartbeat. The radio wave sensor 1 outputs a sensor signal Y1 including the heartbeat information.

Specifically, the radio wave sensor 1 sends out a radio wave as a transmission wave toward a person H1 present within an irradiation area, and receives, as a reception wave, the radio wave reflected from the person H1. Then, the radio wave sensor 1 outputs, based on a differential signal representing the difference between the transmission wave and the reception wave, a sensor signal Y1 including information about the distance between the radio wave sensor 1 and the person H1 or information about the person's H1 motion. Thus, subjecting the sensor signal Y1 to signal processing enables deriving, as the person's H1 biometric information, heartbeat information. The person's H1 heartbeat is one of the person's H1 body motions and may be detected as a variation in the distance between the radio wave sensor 1 and the person H1 or the person's H1 motion. Thus, the heartbeat information is included in the sensor signal Y1 as information about the distance between the radio wave sensor 1 and the person H1 or information about the person's H1 motion.

The radio wave sensor 1 only needs to be a radio wave sensor with the ability to measure the distance to the person H1 or the person's H1 motion. The radio wave sensor 1 may be a frequency-modulated continuous-wave (FMCW) radio wave sensor, a binary frequency shift keying (FSK) radio wave sensor, a doppler radio wave sensor for generating I/Q data, or any other suitable sensor.

The transmission wave sent out from the radio wave sensor 1 is preferably a microwave. In particular, the frequency of the transmission wave preferably falls within either the 24 GHz band or the 48 GHz band. Nevertheless, the transmission wave does not have to be a microwave but may also be a millimeter wave. Thus, the frequency of the transmission wave is not limited to any particular value.

### (3) Signal processing system

The signal processing system 2 includes a computer system. The computer system includes a processor and a memory as principal hardware components thereof. Some or all functions of the signal processing system 2 may be performed by making the processor execute a program stored in the memory. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be implemented as a single or a plurality of electronic circuits including a semiconductor integrated circuit (IC) or a large-scale integrated circuit (LSI). Those electronic circuits may be either integrated together on a single chip or distributed on multiple chips, whichever is appropriate. Those multiple chips may be aggregated together in a single device or distributed in multiple devices without limitation.

Specifically, the signal processing system 2 includes a heartbeat information acquisition unit 21, a representative value calculation unit 23, and a nerve recognition unit 25. The signal processing system 2 preferably further includes a preprocessing unit 22, a storage unit 24, and an output unit 26.

The heartbeat information acquisition unit 21 acquires, from the radio wave sensor 1, a sensor signal Y1 including heartbeat information. The communication between the radio wave sensor 1 and the heartbeat information acquisition unit 21 may be wireless communication or wired communication, whichever is appropriate. The wireless communication is preferably compliant with a protocol such as wireless LAN, Bluetooth^{®}, or ZigBee^{®}. The wired communication is preferably compliant with a wired local area network (LAN) protocol such as Ethernet^{®}. Optionally, the wireless communication and the wired communication may be both compliant with communications protocols dedicated to the signal processing system 2.

The preprocessing unit 22 has an amplification capability of amplifying the sensor signal Y1 and an AD conversion capability of converting the sensor signal Y1 into a digital signal. The preprocessing unit 22 generates a digital sensor signal including a large number of sample values of the sensor signal Y1 generated at a predetermined sampling frequency. Optionally, the radio wave sensor 1 may perform the function of the preprocessing unit 22.

The representative value calculation unit 23 obtains, based on the heartbeat information, a representative value of the person's H1 heartbeat interval (RRI) during a predetermined period. The representative value calculation unit 23 obtains, as a representative value, an average heartbeat interval (average RRI). In particular, the representative value calculation unit 23 preferably obtains, as the average heartbeat interval, a moving average value of the heartbeat interval.

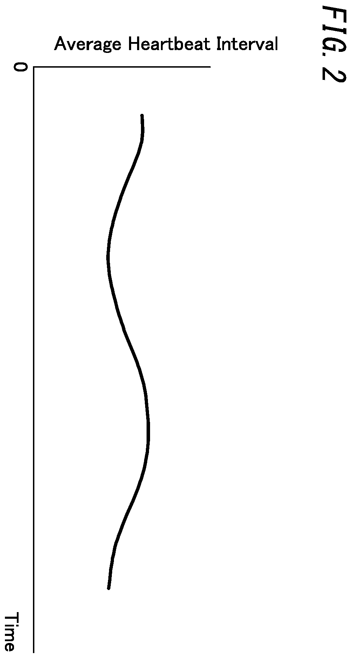

Specifically, the representative value calculation unit 23 detects the timing of one R wave of the heartbeat and obtains a time interval between the R wave and the next R wave, i.e., obtains the person's H1 heartbeat interval. Then, the representative value calculation unit 23 uses a huge number of heartbeat interval data points, which are arranged side by side along the time axis, as time series data of the heartbeat interval. The representative value calculation unit 23 applies a time window to the time series data of the heartbeat interval and extracts a plurality of heartbeat interval data points falling within the time window, as a plurality of extracted data points. The time length of the time window is a predetermined first time (predetermined period). The representative value calculation unit 23 calculates, based on the plurality of extracted data points, a moving average value of the plurality of heartbeat intervals as an average heartbeat interval. The average heartbeat interval calculated by the representative value calculation unit 23 is an average value of the respective heartbeat intervals of the plurality of extracted data points falling within the time window. Then, the representative value calculation unit 23 sequentially obtains, while shifting the time window by a predetermined second time, the average heartbeat intervals every time the second time passes. That is to say, the second time corresponds to a slide time of the window for use to obtain a moving average value of the heartbeat intervals. The representative value calculation unit 23 stores a history of the average heartbeat intervals as time series data of the average heartbeat intervals in the storage unit 24. FIG. 2 shows exemplary time series data of the average heartbeat intervals. Note that the second time is shorter than the first time. For example, if the first time is one minute and the second time is 0.5 seconds, then the average value (average heartbeat interval) of the plurality of heartbeat intervals that cover one minute is obtained every 0.5 second.

The storage unit 24 stores the time series data of the average heartbeat intervals. The storage unit 24 is preferably a programmable nonvolatile memory such as an electrically erasable programmable read-only memory (EEPROM) or a flash memory.

The nerve recognition unit 25 reads out the time series data of the average heartbeat intervals from the storage unit 24. The nerve recognition unit 25 generates frequency domain data of the average heartbeat intervals by subjecting the time series data of the average heartbeat intervals to frequency conversion. Then, the nerve recognition unit 25 obtains, as an index to the condition of the person's H1 autonomic nervous system, the ratio of a low-frequency component of the frequency domain data to a high-frequency component of the frequency domain data.

Specifically, the nerve recognition unit 25 transforms the time series data of the average heartbeat intervals to frequency domain data of the average heartbeat intervals by, for example, maximum entropy method, fast Fourier transform (FFT), or discrete cosine transform (DCT). For example, the nerve recognition unit 25 obtains, as a power spectrum, a distribution of varying frequencies of the average heartbeat intervals. The nerve recognition unit 25 obtains, based on the frequency domain data of the average heartbeat intervals, LF1 as an integral value of signal strengths in a low-frequency domain (from 0.05 Hz to 0.15 Hz, for example) and HF1 as an integral value of signal strengths in a high-frequency domain (from 0.15 Hz to 0.40 Hz, for example). The nerve recognition unit 25 obtains an LF1/HF1 ratio, which is a ratio of LF1 to HF1, as an index to the condition of a person's H1 autonomic nervous system. That is to say, the nerve recognition unit 25 estimates the condition of the person's H1 autonomic nervous system quantitatively by obtaining the LF1/HF1 ratio. The LF1/HF1 ratio when the person's H1 autonomic nervous system is sympathetic dominant is larger than the LF1/HF1 ratio when the person's H1 autonomic nervous system is parasympathetic dominant. For example, the more stressed the person H1 feels, the larger the LF1/HF1 ratio is. The more relaxed the person H1 feels, the smaller the LF1/HF1 ratio is.

The output unit 26 outputs, as a result of recognition made by the nerve recognition unit 25, the LF/HF ratio to the notification system 3. The communication between the output unit 26 and the notification system 3 may be wireless communication or wired communication, whichever is appropriate. The wireless communication is preferably compliant with a protocol such as wireless LAN, Bluetooth^{®}, or ZigBee^{®}. The wired communication is preferably compliant with a wired local area network (LAN) protocol such as Ethernet^{®}. Optionally, the wireless communication and the wired communication may be both compliant with communications protocols dedicated to the signal processing system 2.

The notification system 3 includes at least one of a display device, audio equipment, or any other type of output device. The display device may be, for example, a liquid crystal display or an organic electroluminescent (EL) display and displays the result of recognition made by the nerve recognition unit 25. The audio equipment includes a loudspeaker and outputs, as a voice message, the result of recognition made by the nerve recognition unit 25. The notification system 3 is preferably a personal computer, a tablet computer, a smartphone, or a loudspeaker used by an administrator, for example.

The signal processing system 2 described above recognizes the condition of a person's H1 autonomic nervous system based on a representative value of the heartbeat intervals. The representative value is a representative value of the heartbeat intervals during a predetermined period. The detection accuracy of the heartbeat interval may be lower than that of a high-accuracy heartbeat sensor such as an electrocardiogram detector. In other words, this allows the average heartbeat interval to be kept accurate enough to recognize the condition of a person's H1 autonomic nervous system even without trying to improve the detection accuracy of respective heartbeat intervals.

Thus, the sensor system Z1 according to this embodiment uses the radio wave sensor 1 to detect the heartbeat information that is a result of detection of the person's H1 heartbeat. Then, the signal processing system 2 subjects the sensor signal Y1, supplied from the radio wave sensor 1, to signal processing to obtain a representative value of the person's H1 heartbeat interval and thereby recognize the condition of the person's H1 autonomic nervous system based on the representative value. That is to say, the signal processing system 2 may recognize the condition of the person's H1 autonomic nervous system even without using a high-accuracy heartbeat sensor.

The representative value is preferably a moving average value of the heartbeat intervals (average heartbeat interval) during a predetermined period. In that case, the signal processing system 2 may recognize the condition of a person's H1 autonomic nervous system highly accurately. Note that the representative value does not have to be a moving average value of the heartbeat intervals during the predetermined period but may also be, for example, any one of a median, a maximum value, or a minimum value of the heartbeat intervals during the predetermined period.

### (4) First variation

The sensor system Z1 preferably includes a filter 27 as shown in FIG. 1. The filter 27 has the capability of either filtering out or attenuating an abnormal value of the average heartbeat intervals.

In FIG. 1, the nerve recognition unit 25 includes the filter 27. In this case, the filter 27 either filters out or attenuates data, of which the frequency is equal to or higher than a predetermined frequency threshold value, out of frequency domain data of the average heartbeat intervals. The frequency threshold value may be set at, for example, a value that is a half of the reciprocal of the slide time of the window used by the representative value calculation unit 23 to obtain a moving average value of the heartbeat intervals. The slide time corresponds to a sampling time of the time series data of the average heartbeat interval. The frequency threshold value corresponds to a Nyquist frequency with respect to the sampling frequency of the time series data of the average heartbeat intervals. Thus, the filter 27 may improve the accuracy of the frequency domain data of the average heartbeat interval by either filtering out or attenuating data, of which the frequency is equal to or higher than the Nyquist frequency, out of the frequency domain data of the average heartbeat intervals.

Optionally, the sensor system Z1 may include a filter for either filtering out or attenuating an abnormal value from the time series data of the average heartbeat intervals. The plurality of data points included in the time series data of the average heartbeat intervals are sometimes dispersed. Thus, the plurality of data points may include an abnormal value (outlier) which is significantly different from those of the other data points. Therefore, the filter obtains, by robust estimation, for example, data about an approximation curve based on the time series data of the average heartbeat intervals. Examples of the robust estimation include Huber regression, M-estimation, and random sample consensus (RANSAC). The filter may improve the accuracy of the time series data by obtaining an approximation curve of the time series data by robust estimation. This filter may be provided either between the representative value calculation unit 23 and the storage unit 24 or between the storage unit 24 and the nerve recognition unit 25.

### (5) Second variation

The representative value calculation unit 23 may obtain the heartbeat period as a representative value by subjecting the heartbeat information to frequency conversion.

Specifically, the representative value calculation unit 23 transforms the digital sensor signal generated by the preprocessing unit 22 into frequency domain data of the sensor signal by, for example, maximum entropy method, FFT, or DCT. For example, the representative value calculation unit 23 obtains a power spectrum as frequency domain data of the sensor signal. In the power spectrum, the signal strength reaches a peak at the frequency of the person's H1 heartbeat. Thus, supposing that the person's H1 normal heartbeat frequency is about 60 Hz, the representative value calculation unit 23 extracts a peak in the vicinity of 60 Hz from the power spectrum and obtains a reciprocal of the frequency corresponding to this peak as a representative value of the heartbeat interval. The representative value calculation unit 23 obtains the representative values periodically and stores a history of the representative values as time series data of the representative values in the storage unit 24.

The nerve recognition unit 25 reads out the time series data of the representative values from the storage unit 24. The nerve recognition unit 25 generates frequency domain data of the representative values by subjecting the time series data of the representative values to frequency conversion. Then, the nerve recognition unit 25 obtains a ratio of a low-frequency component of the frequency domain data to a high-frequency component of the frequency domain data as an index to the condition of the person's H1 autonomic nervous system in the same way as described above.

Also, this second variation preferably further includes a filter for either filtering out or attenuating an abnormal value of the representative values as in the first variation described above.

### (6) Biometric management system

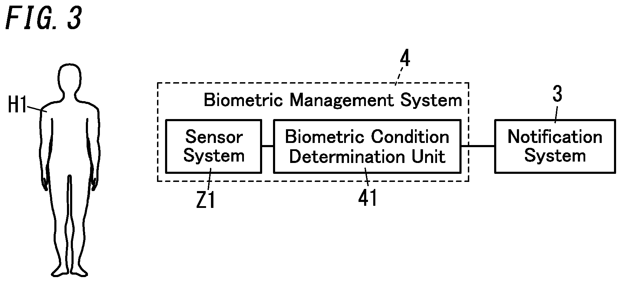

FIG. 3 illustrates a configuration for a biometric management system 4. The biometric management system 4 includes the sensor system Z1 described above and a biometric condition determination unit 41.

The biometric condition determination unit 41 receives a result of recognition made by the nerve recognition unit 25 of the sensor system Z1. The biometric condition determination unit 41 determines, based on the result of recognition made by the nerve recognition unit 25, the person's H1 mental and/or physical condition(s) as a biometric condition.

Note that the biometric condition determination unit 41 may be implemented as either the same computer system as the sensor system Z1 or a different computer system from the sensor system Z1, whichever is appropriate.

The result of recognition made by the nerve recognition unit 25 includes data about the person's H1 LF/HF ratio. This allows the biometric condition determination unit 41 to determine, based on the LF/HF ratio, the person's H1 stress level as a biometric condition. The biometric condition determination unit 41 determines that the larger the LF/HF ratio is, the more stressed out the person's H1 should be (i.e., the smaller the LF/HF ratio is, the less stressed out the person's H1 should be). Optionally, the biometric condition determination unit 41 may determine, based on the LF/HF ratio, not only the person's H1 stress level but also the degree of the person's H1 cognitive function.

The biometric condition determination unit 41 transmits the decision thus made to the notification system 3. In response, the notification system 3 notifies an administrator, for example, of the decision made by the biometric condition determination unit 41. The notification system 3 is preferably a personal computer, tablet computer, or smartphone used by the administrator, for example.

Optionally, the biometric management system 4 may be installed in a moving vehicle steered by the person H1. Examples of the moving vehicle include automobiles, aircrafts, and watercrafts. The moving vehicle is caused to move by being steered by the person H1. In this case, the biometric condition determination unit 41 determines, based on the person's H1 LF/HF ratio, the person's H1 arousal level, thereby making a decision about the person's H1 napping, degree of attention, and/or degree of fatigue as a biometric condition. In that case, the notification system 3 may be a display device and audio equipment in the moving vehicle and is designed to increase the person's H1 arousal level with an image displayed and a sound emitted.

### (7) Environmental control system

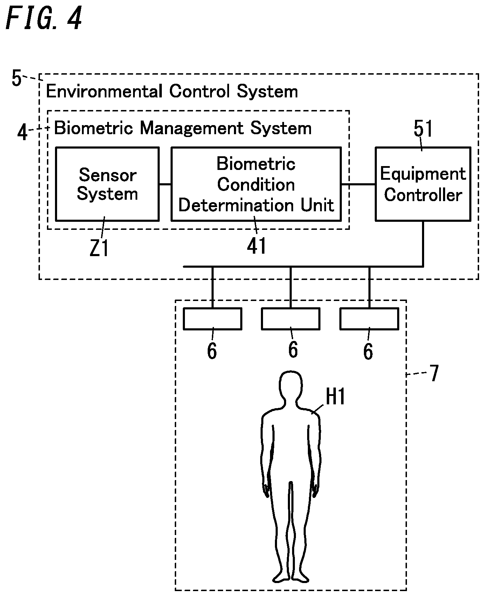

FIG. 4 illustrates a configuration for an environmental control system 5. The environmental control system 5 includes the biometric management system 4 and an equipment controller 51.

The equipment controller 51 receives the decision made by the biometric condition determination unit 41 of the biometric management system 4. The equipment controller 51 controls, based on the decision made by the biometric condition determination unit 41, equipment 6 that changes the environment in a space 7 where the person H1 is present. Note that the equipment controller 51 may be implemented as either the same computer system as the biometric management system 4 or a different computer system from the biometric management system 4, whichever is appropriate.

The decision made by the biometric condition determination unit 41 includes at least one decision made about the person's H1 stress level, the degree of the person's H1 cognitive function, and/or the person's H1 arousal level, for example. Thus, the equipment controller 51 controls, based on the decision made by the biometric condition determination unit 41, the equipment 6 that changes the environment in the space 7. The equipment 6 includes at least one device selected from, for example, the group consisting of an air conditioner, a circulator, a lighting fixture, audio equipment, an aroma diffuser, a display device, a ventilator, and an outside light adjuster (i.e., sunshade). By operating at least one of these pieces of equipment 6, at least one selected from, for example, the group consisting of the temperature, humidity, airflow velocity, illuminance, color of the illuminating light, background music (BGM), aroma, moving picture, CO₂ concentration, and incoming light in the space 7 may be controlled.

Thus, the equipment controller 51 may contribute to, for example, relieving the stress the person H1 feels, improving the person's H1 cognitive function, or increasing the person's H1 arousal level by controlling the equipment 6 based on the decision made by the biometric condition determination unit 41.

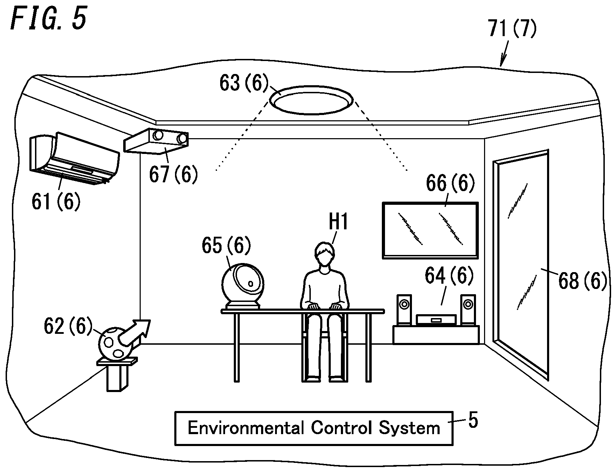

FIG. 5 illustrates, as an exemplary space 7, of which the environment is controlled by the environmental control system 5, a room 71 in which the person H1 is present.

In the room 71, arranged as pieces of equipment 6 are an air conditioner 61, a circulator 62, a lighting fixture 63, audio equipment 64, an aroma diffuser 65, a display device 66, a ventilator 67, and an incoming light adjuster 68. The air conditioner 61 is used to control the temperature and humidity in the room 71. The circulator 62 is used to control the airflow in the room 71. The lighting fixture 63 is used to control the illuminance and light color in the room 71. The audio equipment 64 is used to play background music (BGM) or emit environmental sounds (such as a rain sound, a wind sound, a wave sound, bird's chirrup, and insect's chirping), for example, in the room 71. The aroma diffuser 65 is used to control the aroma in the room 71. The display device 66 is used to display, for example, environmental video (e.g., video of natural scenery such as mountains, oceans, or rivers). The ventilator 67 is used to control the CO₂ concentration in the room 71 by ventilating the room 71. The incoming light adjuster 68 may be an electric blind or an electric curtain, for example, and is used to adjust the amount of outside light entering the room 71. The environmental control system 5 controls, based on the decision made by the biometric management system 4, these pieces of equipment 6, thus controlling the stimulus to the person's H1 visual, aural, olfactory, and tactual senses, for example, and also adjusting the temperature and humidity the person H1 feels in the room 71 and thereby contributing to, for example, relieving the stress the person H1 feels, improving his or her cognitive function, and increasing his or her arousal level.

In particular, the environmental control system 5 may effectively control the person's H1 autonomic nervous system by adjusting the temperature and/or humidity in the room 71 with the air conditioner 61 controlled.

### (8) Signal processing method

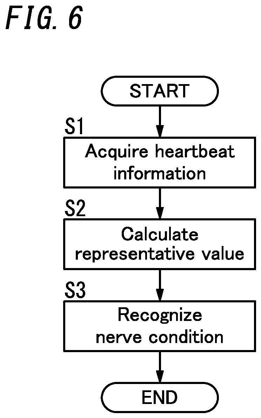

The signal processing method according to this embodiment may be summarized as shown in the flowchart of FIG. 6.

The signal processing method shown in FIG. 6 includes a heartbeat information acquisition step S1, a representative value calculation step S2, and a nerve recognition step S3.

The heartbeat information acquisition step S1 includes making the heartbeat information acquisition unit 21 acquire heartbeat information as a result of detection of a person's H1 heartbeat.

The representative value calculation step S2 includes making the representative value calculation unit 23 calculate, based on the heartbeat information, a representative value of an interval of the heartbeat.

The nerve recognition step S3 includes making the nerve recognition unit 25 recognize, based on the representative value, a condition of the person's H1 autonomic nervous system.

A program stored in a memory of the computer system is preferably designed to cause a processor to perform the signal processing method described above.

The signal processing method and program such as these also enable recognizing the condition of a person's H1 autonomic nervous system even without using a high-accuracy heartbeat sensor.

### (9) Recapitulation

As can be seen from the foregoing description, a signal processing system (2) according to a first aspect of the exemplary embodiment includes a heartbeat information acquisition unit (21), a representative value calculation unit (23), and a nerve recognition unit (25). The heartbeat information acquisition unit (21) acquires, from a radio wave sensor (1), heartbeat information as a result of detection of a person's (HI) heartbeat. The representative value calculation unit (23) calculates, based on the heartbeat information, a representative value of an interval of the heartbeat during a predetermined period. The nerve recognition unit (25) recognizes, based on the representative value, a condition of the person's (HI) autonomic nervous system.

This signal processing system (2) may recognize the condition of a person's (HI) autonomic nervous system even without using a high-accuracy heartbeat sensor.

In a signal processing system (2) according to a second aspect of the exemplary embodiment, which may be implemented in conjunction with the first aspect, the representative value calculation unit (23) calculates, as the representative value, an average value of the interval of the heartbeat.

This signal processing system (2) may recognize the condition of a person's (HI) autonomic nervous system highly accurately.

In a signal processing system (2) according to a third aspect of the exemplary embodiment, which may be implemented in conjunction with the second aspect, the representative value calculation unit (23) calculates, as the average value, a moving average value of the interval of the heartbeat.

This signal processing system (2) may recognize the condition of a person's (HI) autonomic nervous system highly accurately.

In a signal processing system (2) according to a fourth aspect of the exemplary embodiment, which may be implemented in conjunction with the first aspect, the representative value calculation unit (23) preferably calculates, as the representative value, the heartbeat's period by subjecting the heartbeat information to frequency conversion.

This signal processing system (2) may recognize the condition of a person's (HI) autonomic nervous system highly accurately.

A signal processing system (2) according to a fifth aspect of the exemplary embodiment, which may be implemented in conjunction with any one of the first to fourth aspects, preferably further includes a storage unit (24) that stores time series data of the representative value. The nerve recognition unit (25) generates frequency domain data of the representative value by subjecting the time series data of the representative value to frequency conversion and obtains, as the condition of the person's (HI) autonomic nervous system, a ratio of a low-frequency component of the frequency domain data to a high-frequency component of the frequency domain data.

This signal processing system (2) may estimate the condition of a person's (HI) autonomic nervous system quantitatively.

A signal processing system (2) according to a sixth aspect of the exemplary embodiment, which may be implemented in conjunction with any one of the first to fifth aspects, preferably further includes a filter (27) that either filters out or attenuates an abnormal value of the representative value.

This signal processing system (2) may increase the accuracy of average heartbeat interval data.

A sensor system (Z1) according to a seventh aspect of the exemplary embodiment includes the signal processing system (2) according to any one of the first to sixth aspects and a radio wave sensor (1) that receives a radio wave reflected from the person (HI). The radio wave sensor (1) detects the heartbeat information and outputs the heartbeat information to the signal processing system (2).

This sensor system (Z1) may recognize the condition of a person's (HI) autonomic nervous system even without using a high-accuracy heartbeat sensor.

A biometric management system (4) according to an eighth aspect of the exemplary embodiment includes the sensor system (Z1) according to the seventh aspect and a biometric condition determination unit (41) that determines, based on a result of recognition made by the nerve recognition unit (25), the person's (HI) mental and/or physical condition(s) as a biometric condition.

This biometric management system (4) may recognize not only the condition of a person's (HI) autonomic nervous system but also his or her biometric condition as well, even without using a high-accuracy heartbeat sensor.

In a biometric management system (4) according to a ninth aspect of the exemplary embodiment, which may be implemented in conjunction with the eighth aspect, the biometric condition determination unit (41) preferably determines, as the biometric condition, the person's (HI) stress level.

This biometric management system (4) may determine the person's (HI) stress level easily.

In a biometric management system (4) according to a tenth aspect of the exemplary embodiment, which may be implemented in conjunction with the eighth aspect, the biometric condition determination unit (41) preferably determines the biometric condition of the person (HI) who is steering a moving vehicle.

This biometric management system (4) may easily determine the arousal level of the person (HI) who is steering a moving vehicle.

An environmental control system (5) according to an eleventh aspect of the exemplary embodiment includes the biometric management system (4) according to any one of the eighth to tenth aspects and an equipment controller (51) that controls, based on a decision made by the biometric condition determination unit (41), equipment (6) for changing an environment in a space (7) where the person (HI) is present.

This environmental control system (5) may recognize not only the condition of a person's (HI) autonomic nervous system but also his or her biometric condition as well, even without using a high-accuracy heartbeat sensor.

A signal processing method according to a twelfth aspect of the exemplary embodiment includes a heartbeat information acquisition step (S1), a representative value calculation step (S2), and a nerve recognition step (S3). The heartbeat information acquisition step (S1) includes acquiring, from a radio wave sensor (1), heartbeat information as a result of detection of a person's (HI) heartbeat. The representative value calculation step (S2) includes calculating, based on the heartbeat information, a representative value of an interval of the heartbeat during a predetermined period. The nerve recognition step (S3) includes recognizing, based on the representative value, a condition of the person's (HI) autonomic nervous system.

This signal processing method enables recognizing the condition of a person's (HI) autonomic nervous system even without using a high-accuracy heartbeat sensor.

A program according to a thirteenth aspect of the exemplary embodiment is designed to cause a computer system to perform the signal processing method according to the twelfth aspect.

This program enables recognizing the condition of a person's (HI) autonomic nervous system even without using a high-accuracy heartbeat sensor.

A signal processing method according to a fourteenth aspect of the exemplary embodiment includes a heartbeat information acquisition step, a representative value calculation step, and a nerve recognition step. The heartbeat information acquisition step includes acquiring, from a radio wave sensor (1), heartbeat information as a result of detection of a person's (HI) heartbeat. The representative value calculation step includes calculating, based on the heartbeat information, a representative value of an interval of the heartbeat. The nerve recognition step includes obtaining, based on the representative value, an index to a condition of the person's (HI) autonomic nervous system.

This signal processing method enables obtaining an index to the condition of a person's (HI) autonomic nervous system even without using a high-accuracy heartbeat sensor.

### Reference Signs List

- 1: Radio Wave Sensor
- 2: Signal Processing System
- 21: Heartbeat Information Acquisition Unit
- 23: Representative Value Calculation Unit
- 24: Storage Unit
- 25: Nerve Recognition Unit
- 27: Filter
- 4: Biometric Management System
- 41: Biometric Condition Determination Unit
- 5: Environmental Control System
- 51: Equipment Controller
- 6: Equipment
- 7: Space
- H1: Person
- Z1: Sensor System
- S1: Heartbeat Information Acquisition Step
- S2: Representative Value Calculation Step
- S3: Nerve Recognition Step

## Claims

1. A signal processing system comprising:
a heartbeat information acquisition unit configured to acquire, from a radio wave sensor, heartbeat information as a result of detection of a person's heartbeat;
a representative value calculation unit configured to calculate, based on the heartbeat information, a representative value of an interval of the heartbeat during a predetermined period; and
a nerve recognition unit configured to recognize, based on the representative value, a condition of the person's autonomic nervous system.

2. The signal processing system of claim 1, wherein
the representative value calculation unit is configured to calculate, as the representative value, an average value of the interval of the heartbeat.

3. The signal processing system of claim 2, wherein
the representative value calculation unit is configured to calculate, as the average value, a moving average value of the interval of the heartbeat.

4. The signal processing system of claim 1, wherein
the representative value calculation unit is configured to calculate, as the representative value, the heartbeat's period by subjecting the heartbeat information to frequency conversion.

5. The signal processing system of any one of claims 1 to 4, further comprising a storage unit configured to store time series data of the representative value, wherein
the nerve recognition unit is configured to generate frequency domain data of the representative value by subjecting the time series data of the representative value to frequency conversion and obtain, as the condition of the person's autonomic nervous system, a ratio of a low-frequency component of the frequency domain data to a high-frequency component of the frequency domain data.

6. The signal processing system of any one of claims 1 to 5, further comprising a filter configured to filter out or attenuate an abnormal value of the representative value.

7. A sensor system comprising:
the signal processing system of any one of claims 1 to 6; and
a radio wave sensor configured to receive a radio wave reflected from the person,
the radio wave sensor being configured to detect the heartbeat information and output the heartbeat information to the signal processing system.

8. A biometric management system comprising:
the sensor system of claim 7; and
a biometric condition determination unit configured to determine, based on a result of recognition made by the nerve recognition unit, the person's mental and/or physical condition(s) as a biometric condition.

9. The biometric management system of claim 8, wherein
the biometric condition determination unit is configured to determine, as the biometric condition, the person's stress level.

10. The biometric management system of claim 8, wherein
the biometric condition determination unit is configured to determine the biometric condition of the person who is steering a moving vehicle.

11. An environmental control system comprising:
the biometric management system of any one of claims 8 to 10; and
an equipment controller configured to control, based on a decision made by the biometric condition determination unit, equipment configured to change an environment in a space where the person is present.

12. A signal processing method comprising:
a heartbeat information acquisition step including acquiring heartbeat information as a result of detection of a person's heartbeat;
a representative value calculation step including calculating, based on the heartbeat information, a representative value of an interval of the heartbeat during a predetermined period; and
a nerve recognition step including recognizing, based on the representative value, a condition of the person's autonomic nervous system.

13. A program designed to cause a computer system to perform the signal processing method of claim 12.
